# EUROPEAN PATENT APPLICATION

(11) **EP 4 245 865 A1**
(43) Date of publication of application: **20.09.2023**
(21) Application number: 21892409.0
(22) Date of filing: 16.11.2021
(51) Int. Cl.: C12Q 1/6886, G01N 33/68, G01N 33/574

(54) **BIOMARKER FOR PREDICTING THERAPEUTIC RESPONSIVENESS TO CANCER IMMUNOTHERAPEUTIC AGENT AND USE THEREOF**

(30) Priority: 16.11.2020 KR 20200152567
(71) Applicant: Immuneoncia Therapeutics, Inc., Yongin-si, Gyeonggi-do 17084 (KR)
(72) Inventor: CHANG, Sook Kyung, Anyang-si, Gyeonggi-do 14102 (KR); KIM, Jeong Kook, Seongnam-si, Gyeonggi-do 13487 (KR); PARK, Ji Hyun, Suwon-si, Gyeonggi-do 16707 (KR); SHIN, Hee Wook, Seongnam-si, Gyeonggi-do 13487 (KR); SONG, Sun Kwang, Seongnam-si, Gyeonggi-do 13487 (KR)
(74) Representative: Dehns
(86) International application number: PCT/KR2021/016785
(87) International publication number: WO 2022/103242

(57) **Abstract**

The present invention relates to a method for predicting therapeutic responsiveness of a cancer patient to a cancer immunotherapeutic agent, a composition for predicting therapeutic responsiveness, and a kit for predicting therapeutic responsiveness, whereby discrimination can be made between patient groups for which a cancer immunotherapeutic agent is effective and ineffective, in advance, thus greatly enhancing the efficiency of the anticancer therapy using the cancer immunotherapeutic agent.

### Sequence Listing Free Text

<110>	Immune Oncia Therapeutics Inc.

<120>	BIOMARKERS FOR PREDICTING TREATMENT RESPONSE OF
CANCER
	IMMUNOTHERAPY AND USES THEREOF

<130>	FC21531/PCT

<160>	10

<170>	KoPatentIn 3.0

<210>	1

<211>	121

<212>	PRT

<213>	Artificial Sequence

<220>

<223>	heavy chain (VH) variable domain of anti-PD-L1 antibody

<400>  1

Gln Met Gln Leu Val Gln Ser Gly Ala Glu Val Lys Lys Pro Gly Ser

1               5                   10                  15

Ser Val Lys Val Ser Cys Lys Ala Ser Gly Gly Thr Phe Ser Ser Tyr

            20                  25                  30

Ala Tyr Ser Trp Val Arg Gln Ala Pro Gly Gln Gly Leu Glu Trp Met

        35                  40                  45

Gly Gly Ile Ile Pro Ser Phe Gly Thr Ala Asn Tyr Ala Gln Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Pro Ile Val Ala Thr Ile Thr Pro Leu Asp Tyr Trp Gly
            100                 105                 110

Gln Gly Thr Leu Val Thr Val Ser Ser
        115                 120
<210> 2
<211> 108
<212> PRT
<213> Artificial Sequence
<220>
<223> light chain (VL) variable domain of anti-PD-L1 antibody

<400>	2

Ser Tyr Val Leu Thr Gln Pro Pro Ser Val Ser Val Ala Pro Gly Lys

1               5                   10                  15

Thr Ala Thr Ile Ala Cys Gly Gly Glu Asn Ile Gly Arg Lys Thr Val

            20                  25                  30

His Trp Tyr Gln Gln Lys Pro Gly Gln Ala Pro Val Leu Val Ile Tyr

        35                  40                  45

Tyr Asp Ser Asp Arg Pro Ser Gly Ile Pro Glu Arg Phe Ser Gly Ser

    50                  55                  60
Asn Ser Gly Asn Thr Ala Thr Leu Thr Ile Ser Arg Val Glu Ala Gly
65                  70                  75                  80
Asp Glu Ala Asp Tyr Tyr Cys Leu Val Trp Asp Ser Ser Ser Asp His
                85                  90                  95
Arg Ile Phe Gly Gly Gly Thr Lys Leu Thr Val Leu
            100                 105

<210>	3

<211>	470

<212>	PRT

<213>	Artificial Sequence

<220>

<223>	heavy chain of anti-PD-L1 antibody

<400>	3

Met Glu Trp Ser Trp Val Phe Leu Phe Phe Leu Ser Val Thr Thr Gly

1               5                   10                  15

Val His Ser Gln Met Gln Leu Val Gln Ser Gly Ala Glu Val Lys Lys
            20                  25                  30
Pro Gly Ser Ser Val Lys Val Ser Cys Lys Ala Ser Gly Gly Thr Phe
        35                  40                  45
Ser Ser Tyr Ala Tyr Ser Trp Val Arg Gln Ala Pro Gly Gln Gly Leu
    50                  55                  60
Glu Trp Met Gly Gly Ile Ile Pro Ser Phe Gly Thr Ala Asn Tyr Ala
65                  70                  75                  80

Gln Lys Phe Gln Gly Arg Val Thr Ile Thr Ala Asp Glu Ser Thr Ser
                85                  90                  95
Thr Ala Tyr Met Glu Leu Ser Ser Leu Arg Ser Glu Asp Thr Ala Val
            100                 105                 110
Tyr Tyr Cys Ala Arg Gly Pro Ile Val Ala Thr Ile Thr Pro Leu Asp
        115                 120                 125
Tyr Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160
Gly Thr Ala Ala Leu Gly Cys Leu Val Lys Asp Tyr Phe Pro Glu Pro
                165                 170                 175
Val Thr Val Ser Trp Asn Ser Gly Ala Leu Thr Ser Gly Val His Thr
            180                 185                 190
Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220
Val Asn His Lys Pro Ser Asn Thr Lys Val Asp Lys Arg Val Glu Pro
225                 230                 235                 240
Lys Ser Cys Asp Lys Thr His Thr Cys Pro Pro Cys Pro Ala Pro Glu
                245                 250                 255
Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285
Val Ser His Glu Asp Pro Glu Val Lys Phe Asn Trp Tyr Val Asp Gly
    290                 295                 300
Val Glu Val His Asn Ala Lys Thr Lys Pro Arg Glu Glu Gln Tyr Asn
305                 310                 315                 320
Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350
Ala Pro Ile Glu Lys Thr Ile Ser Lys Ala Lys Gly Gln Pro Arg Glu
        355                 360                 365
Pro Gln Val Tyr Thr Leu Pro Pro Ser Arg Glu Glu Met Thr Lys Asn
    370                 375                 380
Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415
Thr Pro Pro Val Leu Asp Ser Asp Gly Ser Phe Phe Leu Tyr Ser Lys
            420                 425                 430
Leu Thr Val Asp Lys Ser Arg Trp Gln Gln Gly Asn Val Phe Ser Cys
        435                 440                 445
Ser Val Met His Glu Ala Leu His Asn His Tyr Thr Gln Lys Ser Leu
    450                 455                 460

Ser Leu Ser Pro Gly Lys
465                    470
<210>    4
<211>    234
<212>    PRT
<213>    Artificial Sequence
<220>
<223>    light chain of anti-PD-L1 antibody

<400>	4

Met Ser Val Pro Thr Gln Val Leu Gly Leu Leu Leu Leu Trp Leu Thr

1               5                   10                  15

Asp Ala Arg Cys Ser Tyr Val Leu Thr Gln Pro Pro Ser Val Ser Val

            20                  25                  30

Ala Pro Gly Lys Thr Ala Thr Ile Ala Cys Gly Gly Glu Asn Ile Gly

        35                  40                  45

Arg Lys Thr Val His Trp Tyr Gln Gln Lys Pro Gly Gln Ala Pro Val
    50                  55                  60
Leu Val Ile Tyr Tyr Asp Ser Asp Arg Pro Ser Gly Ile Pro Glu Arg
65                  70                  75                  80
Phe Ser Gly Ser Asn Ser Gly Asn Thr Ala Thr Leu Thr Ile Ser Arg
                85                  90                  95
Val Glu Ala Gly Asp Glu Ala Asp Tyr Tyr Cys Leu Val Trp Asp Ser
            100                 105                 110

Ser Ser Asp His Arg Ile Phe Gly Gly Gly Thr Lys Leu Thr Val Leu
        115                 120                 125
Gly Gln Pro Lys Ala Ala Pro Ser Val Thr Leu Phe Pro Pro Ser Ser
    130                 135                 140
Glu Glu Leu Gln Ala Asn Lys Ala Thr Leu Val Cys Leu Ile Ser Asp
145                 150                 155                 160
Phe Tyr Pro Gly Ala Val Thr Val Ala Trp Lys Ala Asp Ser Ser Pro
                165                 170                 175

Val Lys Ala Gly Val Glu Thr Thr Thr Pro Ser Lys Gln Ser Asn Asn
            180                 185                 190
Lys Tyr Ala Ala Ser Ser Tyr Leu Ser Leu Thr Pro Glu Gln Trp Lys
        195                 200                 205
Ser His Arg Ser Tyr Ser Cys Gln Val Thr His Glu Gly Ser Thr Val
    210                 215                 220
Glu Lys Thr Val Ala Pro Thr Glu Cys Ser
225                 230

<210> 5

<211> 5

<212> PRT

<213> Artificial Sequence

<220>

<223> CDR1 (VH) of anti-PD-L1 antibody

<400> 5

Ser Tyr Ala Tyr Ser

1                   5

<210>       6

<211>       17

<212>       PRT

<213>       Artificial Sequence

<220>

<223>       CDR2 (VH) of anti-PD-L1 antibody

<400>	6

Gly Ile Ile Pro Ser Phe Gly Thr Ala Asn Tyr Ala Gln Lys Phe Gln

1               5                   10                  15

Gly

<210>	7

<211>	12

<212>	PRT

<213>	Artificial Sequence

<220>

<223>	CDR3 (VH) of anti-PD-L1 antibody

<400>	7

Gly Pro Ile Val Ala Thr Ile Thr Pro Leu Asp Tyr

1               5                   10

<210>	8

<211>	11

<212>	PRT

<213>	Artificial Sequence

<220>

<223>	CDR1 (VL) of anti-PD-L1 antibody

<400>	8

Gly Gly Glu Asn Ile Gly Arg Lys Thr Val His

1               5                   10

<210> 9

<211> 7

<212> PRT

<213> Artificial Sequence

<220>

<223> CDR2 (VL) of anti-PD-L1 antibody

<400> 9

Tyr Asp Ser Asp Arg Pro Ser

1               5

<210> 10

<211> 11

<212> PRT

<213> Artificial Sequence

<220>

<223> CDR3 (VL) of anti-PD-L1 antibody

<400> 10

Leu Val Trp Asp Ser Ser Ser Asp His Arg Ile
1               5                   10

## Description

### Technical Field

The present disclosure relates to a biomarker for predicting therapeutic responsiveness to a cancer immunotherapy and a use thereof.

### Background Art

Conventional cancer treatments were methods of removing cancer cells from a patient as much as possible with surgery, radiation therapy, and chemotherapy. However, surgery and radiation therapy had a therapeutic effect only when cancer cells were completely removed in a case where cancer is at a relatively early stage and has not metastasized. Chemotherapy can be widely used for cancer treatment; however, it had disadvantages of poor cure rate and several adverse effects in most solid cancers. In order to improve these conventional problems, immunotherapy using a cancer immunotherapy has recently emerged.

Among representative cancer immunotherapies, there are antibodies that block the PD-1/PD-L1 pathway, such as anti-PD-1 antibodies and anti-PD-L1 antibodies. These antibodies showed excellent *in vivo* persistence and good clinical effects in a significant number of patients with highly refractory cancer. However, although a significant number of cancer patients show an anticancer effect in response to treatment that blocks the PD-1/PD-L1 pathway, there is a problem in that the treatment does not cause any clinical effect in many patients. Therefore, for the patients who do not respond effectively to treatment using an anti-PD-1/PD-L1 immunotherapy, they lose money and time due to such treatment.

Accordingly, there is a need for a method capable of predicting well whether a tumor responds to treatment using a cancer immunotherapy and selecting a patient group that responds effectively to a specific treatment method; and it is expected that development of such a method greatly contributes to increased survival rate and treatment efficiency in patients.

### Disclosure of Invention

### Technical Problem

An object of the present disclosure is to solve the problems of the prior art as described above.

An object of the present disclosure is to provide a biomarker, a composition, a kit, and a method for providing information, each of which is capable of predicting therapeutic responsiveness to a cancer immunotherapy.

### Solution to Problem

In order to achieve the above-described objects, the present inventors have made many efforts to develop a biomarker capable of predicting therapeutic responsiveness to a cancer immunotherapy, for example, an immune checkpoint inhibitor such as an anti-PD-L1 antibody, an anti-PD-1 antibody, or an anti-CTLA-4 antibody, preferably an anti-PD-L1 antibody. As a result, the present inventors have identified a biomarker, whose expression level varies depending on the presence or degree of cancer treatment effects following administration of a cancer immunotherapy, thereby completing the present disclosure.

According to an aspect of the present disclosure, there is provided a use of an expression level of mRNA or protein of one or more genes selected from the group consisting of OPN, POSTN, and IGFBP-3, for predicting therapeutic responsiveness of a cancer patient to a cancer immunotherapy.

According to another aspect of the present disclosure, there is provided a composition for predicting therapeutic responsiveness of a cancer patient to a cancer immunotherapy, comprising an agent capable of measuring an expression level of mRNA or protein of one or more genes selected from the group consisting of OPN, POSTN, and IGFBP-3.

According to yet another aspect of the present disclosure, there is provided a kit for predicting therapeutic responsiveness of a cancer patient to a cancer immunotherapy, comprising the composition.

According to still yet another aspect of the present disclosure, there is provided a method for providing information for predicting therapeutic responsiveness of a cancer patient to a cancer immunotherapy, comprising measuring an expression level of mRNA or protein of one or more genes selected from the group consisting of OPN, POSTN, and IGFBP-3 in a biological sample obtained from a cancer patient; and evaluating the patient's therapeutic responsiveness to the cancer immunotherapy based on the measured expression level of mRNA or protein.

According to still yet another aspect of the present disclosure, there is provided a method for treating cancer based on therapeutic responsiveness of a cancer patient to a cancer immunotherapy, the method comprising (i) administering the cancer immunotherapy to the cancer patient; and (ii) measuring an expression level of mRNA or protein of one or more genes selected from the group consisting of OPN, POSTN, and IGFBP-3 in a biological sample obtained from the cancer patient before, after, or both before and after administration of the cancer immunotherapy.

### Advantageous Effects of Invention

The present inventors analyzed levels of soluble factors in biological samples isolated from various cancer patients before and after administration of cancer immunotherapies to the patients. As a result, the present inventors identified that expression levels of POSTN, OPN, and IGFBP-3 in serum are significant indicators for predicting therapeutic responsiveness to the cancer immunotherapies.

Use of the biomarker for predicting therapeutic responsiveness to a cancer immunotherapy, according to the present disclosure, makes it possible to distinguish between a patient group that responds effectively to the cancer immunotherapy and a patient group that does not respond effectively to the cancer immunotherapy, in advance or at the beginning of treatment, thereby greatly improving efficiency of anti-cancer treatment using a cancer immunotherapy.

### Brief Description of Drawings

FIG. 1 shows levels of POSTN (periostin) in serum samples from patients before and after treatment with an anti-PD-L1 antibody, according to an embodiment of the present disclosure (nonparametric t test; *, p <0.05; **, p <0.01; ***, p <0.005; ****, p <0.001).
FIG. 2 shows levels of POSTN in serum samples from respective patients.
FIG. 3 shows levels of OPN (osteopontin) in serum samples from patients before and after treatment with an anti-PD-L1 antibody, according to an embodiment of the present disclosure (non-parametric t test; *, p <0.05; **, p <0.01; ***, p <0.005; ****, p <0.001).
FIG. 4 shows levels of OPN in serum samples from respective patients.
FIG. 5 shows levels of IGFBP-3 in serum samples from patients before and after treatment with an anti-PD-L1 antibody, according to an embodiment of the present disclosure (non-parametric t test; *, p <0.05; **, p <0.01; ***, p <0.005).
FIG. 6A shows tumor growth curves for MC38-hPD-L1 tumor (colorectal cancer) in C57BL/6 mice, which are associated with Examples 3.1.1 and 3.2.1.
FIG. 6B shows correlation between serum OPN level and relative tumor volume, wherein the serum was collected on day 21 in a group administered with IMC-001, which is associated with Example 3.1.2. Here, V0 represents a tumor volume on day 0 (before drug administration), and Vt represents a tumor volume on day 21 (after first drug administration).
FIG. 6C shows OPN levels in sera collected on day 21 in a group administered with IMC-001, which are associated with Example 3.1.2. Here, the OPN levels are values measured in two groups divided by relative tumor volume.
FIG. 7A shows tumor growth curves for CT26-hPD-L1/hCD47 tumor (colorectal cancer) in BALB/c mice, which are associated with Examples 3.1.3 and 3.2.3.
FIG. 7B shows correlation between serum OPN level and relative tumor volume, wherein the serum was collected on day 28 in a group administered with IMC-001, which is associated with Example 3.1.4. Here, V0 represents a tumor volume on day 0 (before drug administration), and Vt represents a tumor volume on day 28 (after first drug administration).
FIG. 7C shows OPN levels in sera collected on day 28 in a group administered with IMC-001, which are associated with Example 3.1.4. Here, the OPN levels are values measured in two groups divided by relative tumor volume.
FIG. 8A shows correlation between serum IGFBP-3 level and relative tumor volume, wherein the serum was collected on day 21 in a group administered with IMC-001, which is associated with Example 3.2.2. Here, V0 represents a tumor volume on day 0 (before drug administration), and Vt represents a tumor volume on day 21 (after first drug administration).
FIG. 8B shows IGFBP-3 levels in sera collected on day 21 in a group administered with IMC-001, which are associated with Example 3.2.2. Here, the IGFBP-3 levels are values measured in two groups divided by relative tumor volume.
FIG. 9A shows correlation between serum IGFBP-3 level and relative tumor volume, wherein the serum was collected on day 28 in a group administered with IMC-001, which is associated with Example 3.2.4. Here, V0 represents a tumor volume on day 0 (before drug administration), and Vt represents a tumor volume on day 28 (after first drug administration).
FIG. 9B shows IGFBP-3 levels in sera collected on day 28 in a group administered with IMC-001, which are associated with Example 3.2.4. Here, the IGFBP-3 levels are values measured in two groups divided by relative tumor volume.

### Best Mode for Carrying out Invention

Hereinafter, the present disclosure will be described in detail.

According to an aspect of the present disclosure, there is provided a composition for predicting therapeutic responsiveness of a cancer patient to a cancer immunotherapy, comprising an agent capable of measuring an expression level of mRNA or protein of one or more genes selected from the group consisting of OPN, POSTN, and IGFBP-3.

As used herein, the "predicting therapeutic responsiveness of a cancer patient to a cancer immunotherapy" means predicting whether the cancer patient will respond favorably or unfavorably to the cancer immunotherapy, predicting risk of resistance to the cancer immunotherapy in the cancer patient, or predicting the patient's prognosis, such as recurrence, metastasis, and survival, after such immunotherapy. The composition according to the present disclosure is capable of providing information that allows for selection of the most appropriate immunotherapy modality for a cancer patient. More specifically, the composition according to the present disclosure is capable of predicting whether a patient will respond favorably or unfavorably to a cancer immunotherapy, thereby providing information for facilitating selection of a cancer immunotherapy and increasing therapeutic responsiveness.

As used herein, the term "OPN (osteopontin)" refers to a secreted adhesive glycoprotein of 33 kDa, which is capable of acting in the immune system on regulation of cytokine secretion, cell migration, or the like, or a gene encoding the same. In addition, OPN has been found to be overexpressed in several solid carcinomas, and thus may be a diagnostic marker for various carcinomas. In the present disclosure, OPN may be a biomarker for predicting therapeutic responsiveness to a cancer immunotherapy. For example, the OPN gene, which may be NCBI Gene ID 6696, is a gene belonging to chromosome 4 and may be present in various species. Specifically, the OPN gene may be a polynucleotide consisting of the nucleotide sequence of NCBI Reference Sequence NM_001040058.2.

As used herein, the term "POSTN (periostin)" refers to a 90 kDa protein that regulates cell adhesion and interaction with the extracellular matrix, or a gene encoding the same. POSTN is a protein overexpressed in various epithelial carcinomas such as ovarian cancer and breast cancer, and may be a diagnostic marker for various carcinomas. In the present disclosure, POSTN may be a biomarker for predicting therapeutic responsiveness to a cancer immunotherapy. For example, the POSTN gene, which may be NCBI Gene ID 10631, is a gene belonging to chromosome 13 and may be present in various species. Specifically, the POSTN gene may be a polynucleotide consisting of the nucleotide sequence of NCBI Reference Sequence NM_006475.3.

As used herein, the term "IGFBP-3 (insulin-like growth factor binding protein-3)" refers to one of several proteins, which bind to insulin-like growth factor (IGF)-1 and -2, or a gene encoding the same. Unlike the other IGF-binding proteins, IGFBP-3 has a unique property to associate with an acid-labile subunit (ALS) after binding of either IGF-1 or IGF-2. In addition, IGFBP-3 is known to inhibit growth of breast cancer, prostate cancer, or the like, regardless of IGF, and may be a diagnostic marker for various carcinomas. In the present disclosure, IGFBP-3 may be a biomarker for predicting therapeutic responsiveness to a cancer immunotherapy. For example, the IGFBP-3 gene, which may be NCBI Gene ID: 3486, is a gene belonging to chromosome 7 and may be present in various species. Specifically, the IGFBP-3 gene may be a polynucleotide consisting of the nucleotide sequence of NCBI Reference Sequence NM_001013398.2.

In an embodiment, the one or more genes may comprise OPN.

In an embodiment, the one or more genes may comprise OPN and POSTN.

In an embodiment, the one or more genes may comprise OPN, POSTN, and IGFBP-3.

In the present disclosure, the cancer immunotherapy may be any cancer immunotherapy, such as an immune checkpoint inhibitor, which can be used for anti-cancer immunotherapy.

In an embodiment, the cancer immunotherapy may inhibit binding between PD-L1 and PD-1.

In an embodiment, the cancer immunotherapy may be an anti-PD-1 antibody, an anti-PD-L1 antibody, or an anti-CTLA-4 antibody. Preferably, the cancer immunotherapy may be an anti-PD-L1 antibody. More preferably, for details regarding the anti-PD-L1 antibody, reference may be made to those disclosed in International Publication No. WO2017/0132562. For example, the anti-PD-L1 antibody may comprise a heavy chain variable domain and a light chain variable domain, in which the heavy chain variable domain comprises an amino acid sequence that is at least 95% identical to the amino acid sequence set forth in SEQ ID NO: 1, and the light chain variable domain comprises an amino acid sequence that is at least 95% identical to the amino acid sequence set forth in SEQ ID NO: 2. In addition, the anti-PD-L1 antibody may comprise heavy chain complementarity determining regions (CDRs) having the amino acid sequences set forth in SEQ ID NOs: 5, 6, and 7, and light chain CDRs having the amino acid sequences set forth in SEQ ID NOs: 8, 9, and 10. In addition, the anti-PD-L1 antibody may comprise a heavy chain variable domain having the amino acid sequence set forth in SEQ ID NO: 1 and a light chain variable domain having the amino acid sequence set forth in SEQ ID NO: 2. Further, the anti-PD-L1 antibody may comprise a heavy chain comprising the amino acid sequence set forth in SEQ ID NO: 3 and a light chain comprising the amino acid sequence set forth in SEQ ID NO: 4.

In an embodiment, the agent capable of measuring an expression level of mRNA may be, but is not limited to, an antisense nucleotide, a primer, or a probe which complementarily binds to mRNA of the gene.

As used herein, the term "antisense nucleotide" refers to a nucleic acid-based molecule that has a sequence complementary to a target gene or polynucleotide and thus can form a dimer with the target gene or polynucleotide; and the antisense nucleotide can be used to detect the target gene. The antisense nucleotide may have an appropriate length for its increased detection specificity.

As used herein, the term "primer" refers to a nucleic acid sequence which can form base pairs with a complementary template of a gene or polynucleotide in a sample and serve as a starting point for copying a template strand. A sequence of the primer does not necessarily have to be exactly the same as the template, and the primer only needs to have a sequence sufficiently complementary to the template so that they can hybridize with each other. The primer can initiate DNA synthesis in the presence of a reagent for polymerization and four different nucleoside triphosphates in an appropriate buffer solution and at an appropriate temperature. PCR conditions, and lengths of sense and antisense primers may be modified based on those known in the art.

As used herein, the term "probe" refers to a substance capable of specifically binding to a gene or polynucleotide to be detected in a sample, in which the binding allows to specifically identify the presence of the gene or polynucleotide in the sample. The probe may be prepared in the form of an oligonucleotide probe, a single-stranded DNA probe, a double-stranded DNA probe, or an RNA probe. Selection of suitable probes and hybridization conditions may be modified based on those known in the art.

The antisense nucleotide, the primer, or the probe may be preferably designed using knowledge and techniques known in the art depending on a gene or polynucleotide to be detected.

In an embodiment, the agent capable of measuring an expression level of the protein may be an oligopeptide, an antibody (such as monoclonal antibody, polyclonal antibody, and chimeric antibody), a ligand, a peptide nucleic acid (PNA), or an aptamer, which specifically binds to the protein.

Preferably, the agent capable of measuring an expression level of the protein may be, but is not limited to, an antibody that specifically binds to a protein encoded by the gene.

As used herein, the term "antibody" has the same meaning as is known in the art and may refer to a specific protein molecule directed against an antigenic site. In the present disclosure, the antibody is not particularly limited in its form, and may include polyclonal antibodies, monoclonal antibodies, or parts thereof as long as they have an antigen-binding property, as well as all immunoglobulin antibodies. The antibody may also include special antibodies such as humanized antibodies. For example, the antibody may include a complete form having two full-length light chains and two full-length heavy chains as well as functional fragments of the antibody molecule. The functional fragment of the antibody molecule refers to a fragment that retains at least an antigen-binding function, and examples thereof may include, but are not limited to, Fab, F(ab'), F(ab')2, and Fv.

In an embodiment, the cancer may be a solid cancer. The cancer may be, but is not limited to, one or more selected from the group consisting of cholangiocarcinoma, colon cancer, adenocarcinoma, rectal cancer, breast cancer, synovial sarcoma, chondrosarcoma, thyroid cancer, stomach cancer, thymus cancer, cervical cancer, glioma, brain cancer, melanoma, lung cancer, bladder cancer, prostate cancer, leukemia, kidney cancer, liver cancer, colorectal cancer, pancreatic cancer, ovarian cancer, lymphoma, uterine cancer, oral cancer, bronchial cancer, nasopharyngeal cancer, laryngeal cancer, skin cancer, blood cancer, parathyroid cancer, and ureteral cancer. In addition, the cancer may be colorectal cancer.

According to another aspect of the present disclosure, there is provided a kit for predicting therapeutic responsiveness of a cancer patient to a cancer immunotherapy, comprising the composition. In the kit, the "predicting therapeutic responsiveness of a cancer patient to a cancer immunotherapy" and the "cancer" are as described above.

Specifically, the kit consists of one or more other component compositions, solutions, or devices, which are suitable for assay methods.

In an embodiment, the kit may be a kit for measuring an expression level of mRNA of the gene, for example, a kit containing essential elements to perform RT-PCR. For example, the kit may contain other reagents which are known in the art to be required to perform RT-PCR.

In an embodiment, the kit may be a kit for measuring an expression level of the protein, for example, an enzyme-linked immunosorbent assay (ELISA) kit. The kit may contain other reagents which are known in the art to be required for immunological detection of antibodies.

According to still yet another aspect of the present disclosure, there is provided a method for providing information for predicting therapeutic responsiveness of a cancer patient to a cancer immunotherapy, comprising measuring an expression level of mRNA or protein of one or more genes selected from the group consisting of OPN, POSTN, and IGFBP-3 in a biological sample obtained from the cancer patient; and evaluating the patient's therapeutic responsiveness to the cancer immunotherapy based on the measured expression level of mRNA or protein. In the above-described method, the measuring an expression level of mRNA of gene, the measuring an expression level of protein, and the predicting therapeutic responsiveness of a cancer patient to a cancer immunotherapy are as described above.

As used herein, the term "biological sample" refers to any biological sample obtained from an individual, and examples thereof may include, but are not limited to, a sample (such as blood, serum, plasma, lymph, saliva, or urine) obtained from an individual whose therapeutic responsiveness to a cancer immunotherapy is intended to be determined, for example, a cancer patient.

In an embodiment, the method may further comprise determining whether the patient needs to receive treatment with the cancer immunotherapy or needs to continue treatment with the cancer immunotherapy, depending on the evaluation of the patient's therapeutic responsiveness to the cancer immunotherapy.

In an embodiment, the method may further comprise administering the cancer immunotherapy to the patient. The administering may be performed before, after, or both before and after measuring an expression level of mRNA or protein of the one or more genes. For example, the expression level may be measured immediately before administration of the cancer immunotherapy, and 20 to 60 days, 25 to 50 days, 30 to 45 days, or 35 to 43 days after first administration of the cancer immunotherapy. For example, the therapeutic responsiveness to the cancer immunotherapy may be evaluated by obtaining a blood sample from the cancer patient, measuring an expression level of one or more genes of OPN, POSTN, and IGFBP-3, measuring an expression level of the same gene(s) again 40 days after starting treatment with the cancer immunotherapy, and comparing the expression levels before and after the treatment with the cancer immunotherapy. Here, the therapeutic responsiveness may be evaluated based on an increased or decreased expression level, the pattern of which differs depending on each gene or a protein expressed therefrom.

In addition, the evaluating the patient's therapeutic responsiveness to the cancer immunotherapy based on the measured expression level of mRNA or protein comprises comparing an expression level of the mRNA or protein measured in the cancer patient before (and/or after) administration of the cancer immunotherapy with an expression level of the mRNA or protein measured in other control cancer patients before (and/or after) administration of the cancer immunotherapy. The expression level measured in the other control cancer patients before and/or after administration of the cancer immunotherapy may be an average expression level identified in biological samples obtained from a plurality of control cancer patients, who have been identified to have therapeutic responsiveness to the cancer immunotherapy or have no therapeutic responsiveness to the cancer immunotherapy, before and/or after administration of the cancer immunotherapy.

In an embodiment, the expression level is measured before and after administration of the cancer immunotherapy, wherein when the gene is OPN or POSTN, the patient may be evaluated as having low therapeutic responsiveness in a case where the measured expression level is significantly higher after administration of the cancer immunotherapy than before administration of the cancer immunotherapy. For example, for OPN, in a case where the expression level measured after administration of the cancer immunotherapy is 1.1 to 2 times, 1.2 to 2 times, or 1.5 to 1.8 times the expression level measured before administration of the cancer immunotherapy, the cancer patient may be evaluated as having low therapeutic responsiveness to the cancer immunotherapy. In addition, in a case where the expression level measured after administration of the cancer immunotherapy is 0.4 to 1 times, 0.4 to 0.9 times, or 0.5 to 0.8 times the expression level measured before administration of the cancer immunotherapy, the cancer patient may be evaluated as having high therapeutic responsiveness to the cancer immunotherapy. For example, for POSTN, in a case where the expression level measured after administration of the cancer immunotherapy is 1.1 to 2 times, 1.2 to 2 times, or 1.5 to 1.8 times the expression level measured before administration of the cancer immunotherapy, the cancer patient may be evaluated as having low therapeutic responsiveness to the cancer immunotherapy. In addition, in a case where the expression level measured after administration of the cancer immunotherapy is 0.4 to 1 times or 0.5 to 0.8 times the expression level measured before administration of the cancer immunotherapy, the cancer patient may be evaluated as having high therapeutic responsiveness to the cancer immunotherapy.

In an embodiment, the expression level is measured before or after administration of the cancer immunotherapy, wherein when the gene is OPN or POSTN, the patient may be evaluated as having low therapeutic responsiveness in a case where the measured expression level is significantly higher than the expression level measured in other control cancer patients (for example, patients evaluated as having high therapeutic responsiveness) before or after administration of the cancer immunotherapy.

In an embodiment, the expression level is measured before and after administration of the cancer immunotherapy, wherein when the gene is IGFBP-3, the patient may be evaluated as having low therapeutic responsiveness in a case where the measured expression level is significantly lower after administration of the cancer immunotherapy than before administration of the cancer immunotherapy. For example, in a case where the expression level measured after administration of the cancer immunotherapy is 0.4 to 1 times, 0.4 to 0.9 times, or 0.5 to 0.8 times the expression level measured before administration of the cancer immunotherapy, the cancer patient may be evaluated as having low therapeutic responsiveness to the cancer immunotherapy. For example, in a case where the expression level measured after administration of the cancer immunotherapy is 1.1 to 2 times, 1.2 to 2 times, or 1.5 to 1.8 times the expression level measured before administration of the cancer immunotherapy, the cancer patient may be evaluated as having high therapeutic responsiveness to the cancer immunotherapy.

In an embodiment, the expression level is measured before or after administration of the cancer immunotherapy, wherein when the gene is IGFBP-3, the patient may be evaluated as having low therapeutic responsiveness in a case where the measured expression level is significantly lower than the expression level measured in other control cancer patients (for example, patients evaluated as having high therapeutic responsiveness) before or after administration of the cancer immunotherapy.

In an embodiment, the expression level of the mRNA may be measured by one or more methods selected from the group consisting of in situ hybridization, reverse transcriptase polymerase chain reaction (RT-PCR), competitive reverse transcriptase polymerase chain reaction (competitive RT-PCR), real-time reverse transcriptase polymerase chain reaction (real-time RT-PCR), RNase protection assay (RPA), microarray, and northern blotting, but the method is not limited thereto.

In an embodiment, the expression level of the protein may be measured by one or more methods selected from the group consisting of Western blotting, radioimmunoassay (RIA), radioimmunodiffusion, enzyme-linked immunosorbent assay (ELISA), immunoprecipitation, flow cytometry, immunofluorescence, Ouchterlony, complement fixation assay, and protein chip, but the method is not limited thereto.

According to still yet another aspect of the present disclosure, there is provided a method for treating cancer based on therapeutic responsiveness of a cancer patient to a cancer immunotherapy, the method comprising (i) administering the cancer immunotherapy to the cancer patient; and (ii) measuring an expression level of mRNA or protein of one or more genes selected from the group consisting of OPN, POSTN, and IGFBP-3 in a biological sample obtained from the cancer patient before, after, or both before and after administration of the cancer immunotherapy.

In an embodiment, the method may further comprise evaluating the patient's therapeutic responsiveness to the cancer immunotherapy based on the measured mRNA or protein expression level.

In an embodiment, the method may further comprise determining whether the patient needs to receive treatment with the cancer immunotherapy or needs to continue treatment with the cancer immunotherapy, depending on the evaluation of the patient's therapeutic responsiveness to the cancer immunotherapy.

Regarding the above-described method, the cancer immunotherapy, the gene, the cancer, the biological sample, the measuring an expression level of mRNA, the measuring an expression level of protein, the evaluating therapeutic responsiveness, and the like are as described above.

Hereinafter, the present disclosure will be described in detail by way of examples. However, the following examples are only for illustrating the present disclosure, and the present disclosure is not limited to the following examples.

### Example 1. Materials and methods

### Example 1.1. Information on patient group and collection of sample

Patients with a metastatic solid cancer or a locally advanced solid cancer were subjected to anti-cancer immunotherapy with an anti-PD-L1 antibody (an anti-PD-L1 antibody (IMC-001) comprising a heavy chain that comprises the amino acid sequence set forth in SEQ ID NO: 3 and a light chain that comprises the amino acid sequence set forth in SEQ ID NO: 4), and blood samples were obtained from the patients.

Specifically, intravenous administration of the antibody IMC-001 was performed every 2 weeks according to a standard 3+3 dose escalation design until disease progression or occurrence of unacceptable toxicity. A total of 15 patients (8 males and 7 females, with their average age being 58 years old) were included in 5 dose escalation cohorts (2, 5, 10, 15, and 20 mg/kg).

Blood samples were collected from the patients before initiation of dosing (Day 1), on days 3 and 8 after dosing (Days 3 and 8), and on days 15 and 43 after dosing (Days 15 and 43). The blood samples were subjected to centrifugation and serum samples were collected therefrom. All samples were immediately separated, frozen, and stored at -80°C until use. Type of cancer and BOR information for the blood samples by date of collection are as shown in Table 1.

**[Table 1]**

| Blood sample | Type of cancer | BOR |
|---|---|---|
| Day 1, 3, 8, 15, 43 | Cholangiocarcinoma/liver metastasis | SD |
| Day 1, 3, 8, 15, 43 | Gallbladder adenocarcinoma /lymph metastasis | |
| Day 1, 3, 8, 15, 43 | Cholangiocarcinoma/liver metastasis | |
| Day 1, 3, 8, 15, 43 | Thymus cancer/lung and bone metastasis | |
| Day 1, 3, 8, 15, 43 | Rectal cancer/lymph metastasis | SD→PR |
| Day 1, 3, 8, 15, 43 | Rectal cancer/lung and liver metastasis | PD |
| Day 1, 3, 8, 15, 43 | Thymus cancer/lung and liver metastasis | |
| Day 1, 3, 8, 15, 43 | Thyroid cancer/lung, spleen, and lymph metastasis | NE |
| Day 1, 3, 8, 15 | Colon cancer/liver metastasis | PD |
| Day 1, 3, 8, 15 | Rectal cancer/lung metastasis | |
| Day 1, 3, 8, 15 | Breast cancer | |
| Day 1, 3, 8, 15 | Synovial sarcoma/lung and liver metastasis | |
| Day 1, 3, 8, 15 | Chondrosarcoma/lung and bone metastasis | |
| Day 1, 3, 8, 15 | Stomach cancer/rectum metastasis | |
| Day 1, 3, 8, 15 | Rectal cancer/lung metastasis | |

BOR (best overall response): Best overall response recorded from initiation of dosing until disease progression/relapse; SD: stable disease; PD: progressive disease; PR: partial response; and NE: not evaluable. The total number of patients was 15, in which SD was 4, PR was 1, PD was 9, and NE was 1.

### Example 1.2. Multiplexed assay

The blood samples from the patient group were screened for each of 55 soluble factors by analyzing serum samples using Luminex's xMAP Technology, which is a quantitative multiplexed immunoassay using automated bead-based technology (see Table 2).

**[Table 2]**

| | |
|---|---|
| Circulating tumor marker | Alpha-fetoprotein (AFP) |
| | Fibroblast growth factor 2 (FGF-2) |
| | Human epididymis protein 4 (HE4) |
| | Hepatocyte growth factor (HGF) |
| | Interleukin-6 (IL-6) |
| | Interleukin-8 (IL-8) |
| | Leptin |
| | Macrophage migration inhibitory factor (MIF) |
| | Osteopontin (OPN) |
| | Prolactin |
| | Stem cell factor (SCF) |
| | Tumor growth factor α (TGF- α) |
| | Tumor necrosis factor α (TNF- α) |
| | TNF-related apoptosis inducing ligand (TRAIL) |
| | Vascular endothelial growth factor (VEGF) |
| | Cancer antigen 125 (CA125) |
| | Cancer antigen 15-3 (CA15-3) |
| | Ferritin |
| | Galectin3 |
| | Insulin growth factor binding protein-3 (IGFBP-3) |
| | Myeloperoxidase (MPO) |
| Angiogenic marker | Angiopoietin 2 (Ang2) |
| | Bone morphogenetic protein 9 (BMP-9) |
| | Epidermal growth factor (EGF) |
| | Endoglin |
| | Fibroblast growth factor 1 (FGF-1) |
| | Granulocyte colony stimulating factor (G-CSF) |
| | Heparin-binding epidermal growth factor-like growth factor |
| | (HB-EGF) |
| | Placental growth factor (PLGF) |
| | Vascular endothelial growth factor C (VEGF-C) |
| | Vascular endothelial growth factor (VEGF-D) |
| Metastatic marker | Dickkopf 1 (DKK-1) |
| | Growth differentiation factor 15 (GDF-15) |
| | Periostin (POSTN) |
| Cytokine/chemokine | Interferon γ (IFN-γ) |
| | Interleukin 1α (IL-1α) |
| | Interleukin 1β (IL-1β) |
| | Interleukin 1 receptor antagonist (IL-1rα) |
| | Interleukin 2 (IL-2) |
| | Interleukin 4 (IL-4) |
| | Interleukin 10 (IL-10) |
| | Interleukin 12 (IL-12) |
| | Interleukin 15 (IL-15) |
| | Interleukin 17α (IL-17α) |
| | C-X-C motif ligand 9 (CXCL9) |
| | C-X-C motif ligand 10 (CXCL10) |
| | Platelet-derived growth factor AB (PDGF-AB) |
| | Platelet-derived growth factor BB (PDGF-BB) |
| | Tie-2 |
| | Chemokine ligand 5 (CCL5) |
| | Monocyte chemotactic protein 1 (MCP-1) |
| | Monocyte chemotactic protein 3 (MCP-3) |
| | Chemokine ligand 22(CCL22) |
| | Macrophage inflammatory protein 1 alpha (MIP-1α) |
| | Macrophage inflammatory protein 1 beta (MIP-1β) |

Human premixed multiplex assay kits for fabrication of plex panels were purchased from R&D Systems (Catalog nos. LXSAHM-06, 10, 18, 21). xMAP analysis of serum was performed using Luminex 100 in Koma Biotech Inc. (Seoul, Republic of Korea). Experimental data were analyzed using 5-parameter curve fitting in MasterPlex QT 2010 software.

### Example 2. Results obtained by performing analysis of soluble factors before and after administration of cancer immunotherapy

The analysis results in Example 1.2 were obtained from Koma Biotech Inc., and serum levels of the 55 factors were analyzed to perform comparison between the SD group and the PD group. Analysis of the 55 soluble factors showed that POSTN, OPN, and IGFBP-3 were factors that produced significant experimental results which allow for identification of therapeutic responsiveness to the cancer immunotherapy.

### Example 2.1. POSTN and OPN showed low levels both at baseline and after treatment with anti-PD-L1 antibody in SD group.

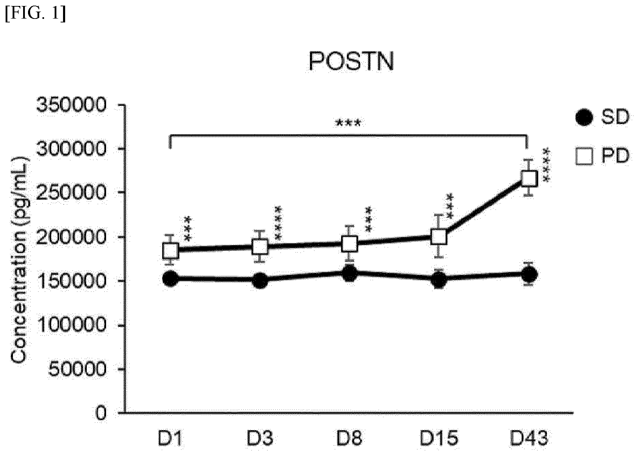

The analysis results for POSTN and OPN are shown in FIGS. 1 to 4.

POSTN (periostin) is an adhesion molecule and OPN (osteopontin) is an intracellularly secreted glycoprotein.

The analysis results showed that the POSTN level in the PD group was higher than that in the SD group at baseline and increased significantly until day 43, whereas the POSTN level in the SD group remained low during the test period (range of average POSTN level in the SD group: 153316.0 to 158397.3 pg/mL, see FIG. 1).

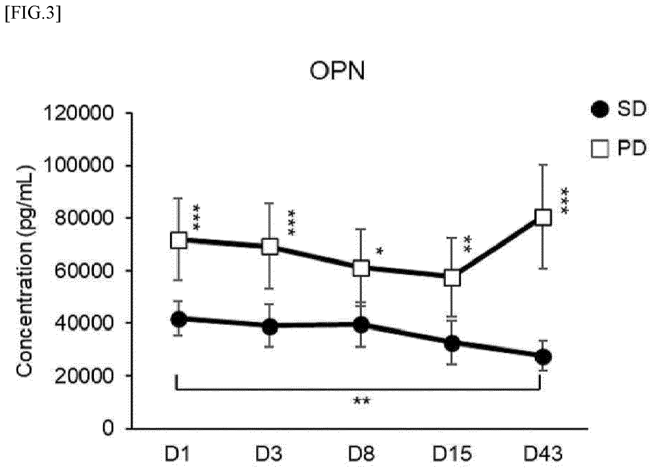

In the SD group, OPN gradually decreased until day 43, whereas in the PD group, OPN remained higher than in the SD group starting from baseline and did not change greatly over time (range of average OPN level in the SD group: 27687 to 41942.3 pg/mL, see FIG. 3).

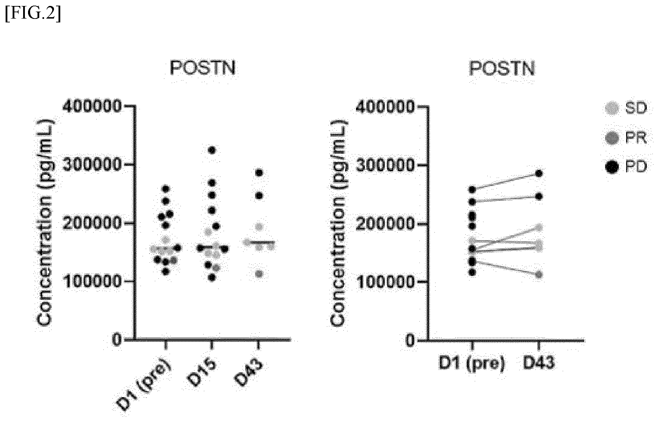

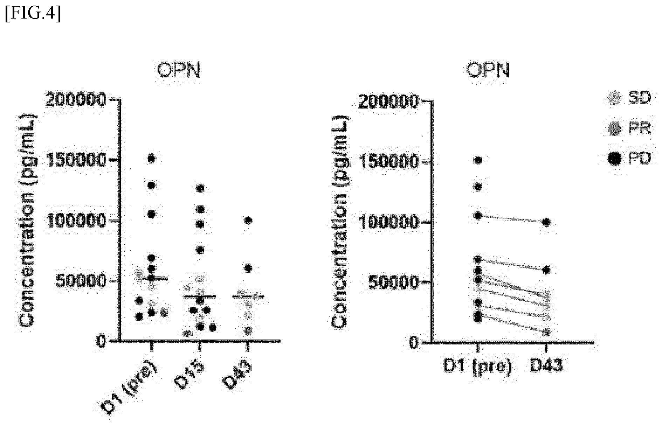

For the samples obtained from the patients whose BOR changed from SD to PR, the levels of POSTN and OPN gradually decreased until day 43 and were at the lowest on day 43 (see FIGS. 2 and 4).

Therefore, for a cancer patient group, in a case where the group shows relatively low baseline levels of POSTN and OPN, or a lowered level of OPN is produced in the group after treatment with the cancer immunotherapy as compared with before treatment with the cancer immunotherapy, the group may be selected as a patient group suitable for the treatment with the cancer immunotherapy.

### Example 2.2. PD group showed low level of IGFBP-3 on day 43 after administration of anti-PD-L1 antibody.

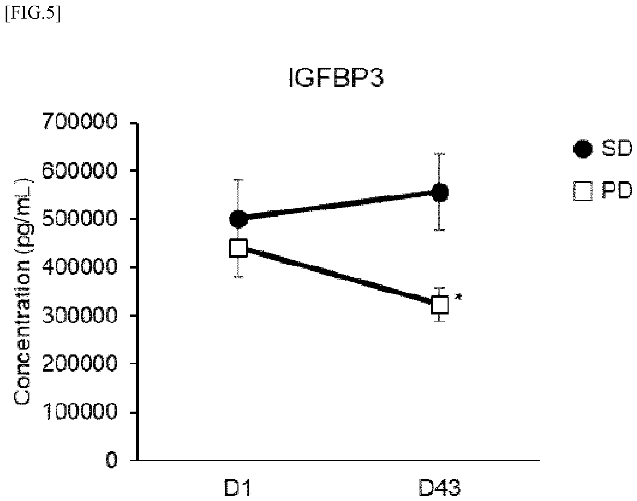

The IGFBP-3 level was not significantly different between the SD and PD groups at baseline. However, the IGFBP-3 level was significantly changed during the course of treatment with the anti-PD-L1 antibody (FIG. 5). After administration of the anti-PD-L1 antibody, the IGFBP-3 level decreased in the sera from the patients in the PD group, whereas before and after the treatment, the IGFBP-3 level slightly increased in the sera from the patients in the SD group.

These results suggest that in a case where a patient shows a low IGFBP3 level after administration of the anti-PD-L1 antibody, IGFBP3 may be a biomarker which indicates that the patient has no responsiveness to the antibody.

### Example 3. Animal experiments using colorectal cancer mouse model

A request for animal experiments using a colorectal cancer mouse model was made to Biocytogen and Gempharmatech. A colorectal cancer mouse model obtained with C57BL/6 mice and MC38-hPD-L1 tumor cells was developed by Biocytogen, and a colorectal cancer mouse model obtained with BALB/c mice and CT26-hPD-L1/hCD47 tumor cells was developed by Gempharmatech.

### Example 3.1. Therapeutic responsiveness following administration of IMC-001 (biomarker: OPN)

### Example 3.1.1. Changes in tumor volume following administration of IMC-001 in MC38-hPD-L1 tumor model

In vivo efficacy of IMC-001 was evaluated for treatment of MC38-hPD-L1 tumor in C57BL/6 mice as a colorectal cancer model.

For tumor development, the C57BL/6 mice were subcutaneously injected on the right anterior flank with 5×10⁵ MC38-hPD-L1 tumor cells suspended in 0.1 mL PBS. The tumor-bearing mice whose average tumor size reached about 100 mm³ were randomly enrolled in the group hIgG 1 20 mg/kg, the group IMC-001 3 mg/kg, and the group IMC-001 10 mg/kg (each group consisting of 8 mice). All test substances were intraperitoneally administered to the tumor-bearing mice at a frequency of 3 times a week for 3 weeks.

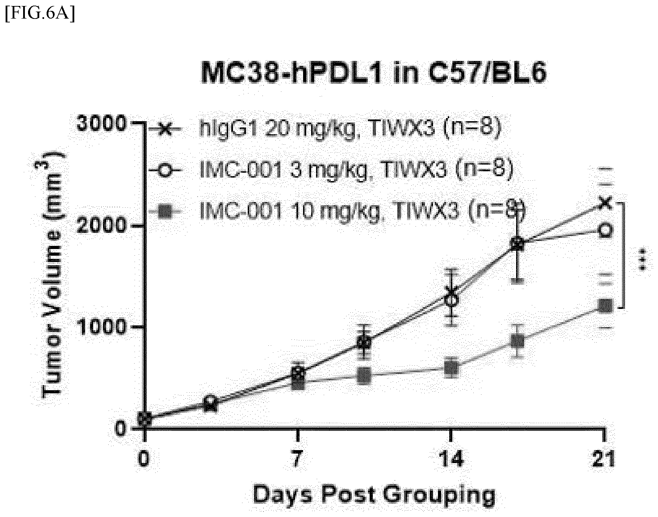

The mice were sacrificed on day 21. GraphPad Prism 8 software was used to plot graphs of tumor volume, and comparison between the groups was performed using two-way ANOVA with Dunnett's multiple comparison test (p-value; * <0.05, ** <0.01, *** < 0.001, **** <0.0001) (FIG. 6A).

### Example 3.1.2. Determination of mouse serum OPN concentration by ELISA

At the time of sacrifice of the mice as in Example 3.1.1, all serum samples were collected and stored at -80°C until analysis.

Mouse serum OPN was measured by enzyme-linked immunosorbent assay (ELISA) using the mouse OPN duoset ELISA kit (DY411, R&D Systems, Minneapolis, Minnesota, United States), and the procedure was performed according to the instructions of the manufacturer of the kit.

All samples were diluted at a ratio of 1:8,000 using 1× reagent diluent concentrate 2 from Duoset Ancillary Reagent Kit 2 (DY008, R&D Systems) within the range of the standard curve. All samples were run in duplicate.

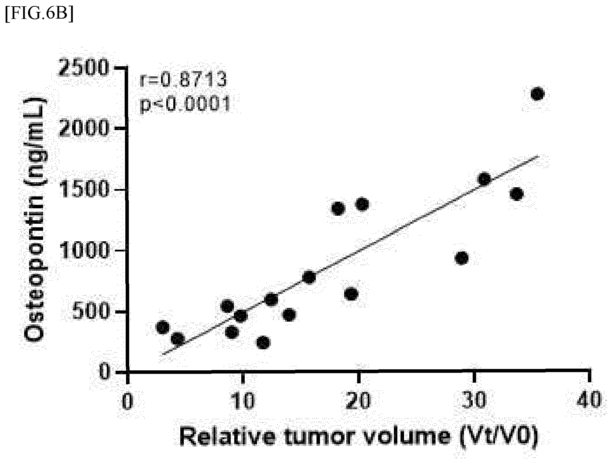

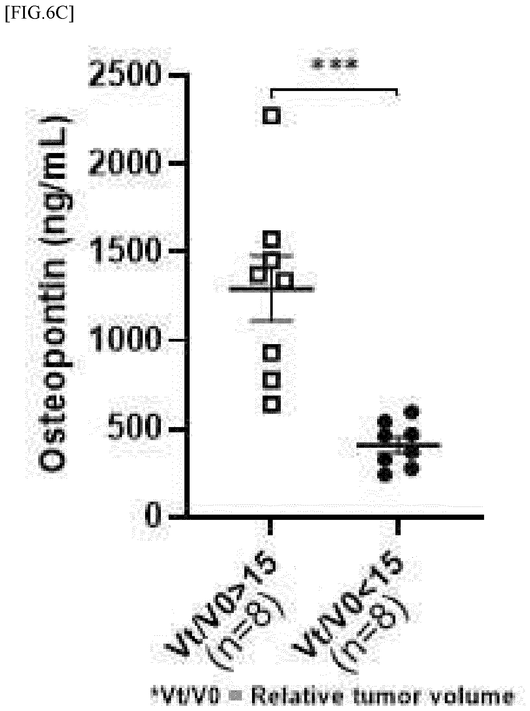

GraphPad Prism 8 software was used to plot variable data, and then correlation between two parameters (serum OPN and relative tumor volume) was identified using Pearson's correlation analysis (FIG. 6B). Then, comparison of differences between two groups was performed using an unpaired t-test, and a p-value of less than 0.05 was considered statistically significant (p-value; * <0.05, ** <0.01, *** <0.001, **** <0.0001) (FIG. 6C). In FIG. 6C, based on the relative tumor volume, the total of 16 mice administered with IMC-001 were divided into two groups of eight (8) each, that is, a group with large tumor growth (group having poor responsiveness (Vt/V0>15), n=8) and a group with small tumor growth (group having good responsiveness (Vt/V0<15), n=8), and OPN was measured.

### Example 3.1.3. Changes in tumor volume following administration of IMC-001 in CT26-hPD-L1/hCD47 tumor model

In vivo efficacy of IMC-001 was evaluated for treatment of CT26-hPD-L1/hCD47 tumor in BALB/c mice as a colorectal cancer model.

For tumor development, the BALB/c mice were subcutaneously injected on the right anterior flank with 2×10⁶ CT26-hPD-L1/hCD47 tumor cells suspended in 0.1 mL PBS. The tumor-bearing mice whose average tumor size reached about 100 mm³ were randomly enrolled in the group hIgG 1 21.4 mg/kg, the group IMC-001 2.1 mg/kg, and the group IMC-001 10.7 mg/kg (each group consisting of 8 mice). All test substances were intraperitoneally administered to the tumor-bearing mice at a frequency of twice a week for the first 2 weeks, and tumor volume was measured for 2 more weeks.

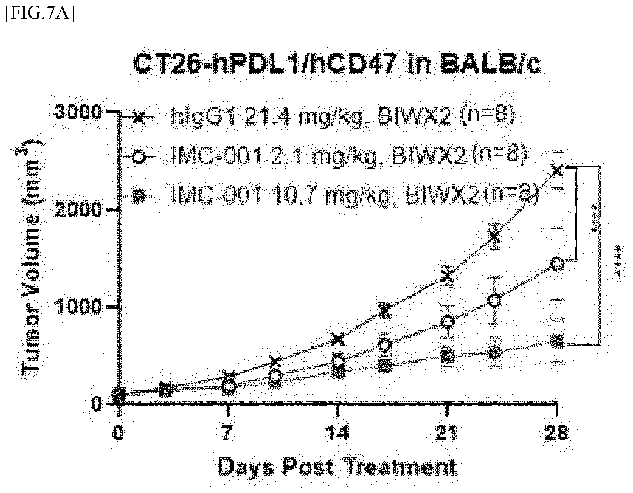

The mice were sacrificed on day 28 after the first administration of IMC-001. GraphPad Prism 8 software was used to plot graphs of tumor volume, and comparison between the groups was performed using two-way ANOVA with Dunnett's multiple comparison test (p-value; * <0.05, ** <0.01, *** < 0.001, **** <0.0001) (FIG. 7A).

### Example 3.1.4. Determination of mouse serum OPN concentration by ELISA

At the time of sacrifice of the mice as in Example 3.1.3, all serum samples were collected and stored at -80°C until analysis.

Mouse serum OPN was measured by enzyme-linked immunosorbent assay (ELISA) using the mouse OPN duoset ELISA kit (DY411, R&D Systems, Minneapolis, Minnesota, United States), and the procedure was performed according to the instructions of the manufacturer of the kit. All samples were diluted at a ratio of 1:8,000 using 1× reagent diluent concentrate 2 from Duoset Ancillary Reagent Kit 2 (DY008, R&D Systems) within the range of the standard curve. All samples were run in duplicate.

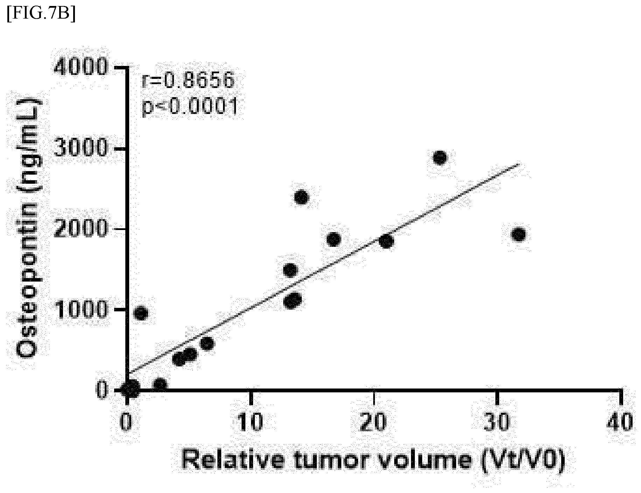

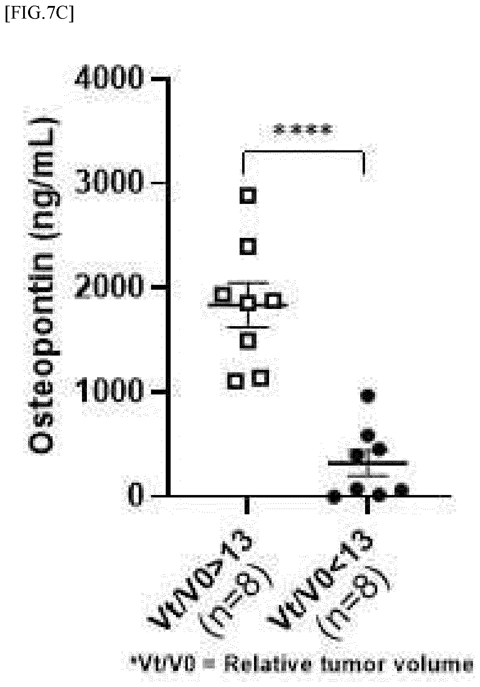

GraphPad Prism 8 software was used to plot variable data, and then correlation between two parameters (serum OPN and relative tumor volume) was identified using Pearson's correlation analysis (FIG. 7B). Then, comparison of differences between two groups was performed using an unpaired t-test, and a p-value of less than 0.05 was considered statistically significant (p-value; * <0.05, ** <0.01, *** <0.001, **** <0.0001) (FIG. 7C). In FIG. 7C, based on the relative tumor volume, the total of 16 mice administered with IMC-001 were divided into two groups of eight (8) each, that is, a group with large tumor growth (group having poor responsiveness (Vt/V0>13), n=8) and a group with small tumor growth (group having good responsiveness (Vt/V0<13), n=8), and OPN was measured.

### Example 3.1.5. Results

In the two colorectal cancer mouse models (MC38 and CT26) used in Examples 3.1.1 to 3.1.4, tumor growth was inhibited in the groups administered with IMC-001 as compared with each control group (hIgG1) (FIGS. 6A and 7A).

In addition, as shown in FIG. 6C (average OPN value for Vt/V0> 15: 1294.8 ng/mL (SEM value: 184.3); average OPN value for Vt/V0<15: 409.8 ng/mL (SEM value: 44.8)) and FIG. 7C (average OPN value for Vt/V0>13: 1836.6 ng/mL (SEM value: 214.4); average OPN value for Vt/V0<13: 319.5 ng/mL (SEM value: 121.7)), in the groups (n=16) administered with IMC-001, serum OPN level was significantly lower in the group with small tumor growth (group having good responsiveness, n=8) than the group with large tumor growth (group having poor responsiveness, n=8).

From these results, since in the groups administered with IMC-001, a lower OPN level was observed as a relative tumor volume was smaller, it was identified that OPN could be used as a biomarker to predict whether an anti-PD-L1 antibody such as IMC-001 would exert a therapeutic effect.

### Example 3.2. Therapeutic responsiveness following administration of IMC-001 (biomarker: IGFBP-3)

### Example 3.2.1. Changes in tumor volume following administration of IMC-001 in MC38-hPD-L1 tumor model

In vivo efficacy of IMC-001 was evaluated for treatment of MC38-hPD-L1 tumor in C57BL/6 mice as a colorectal cancer model.

For tumor development, the C57BL/6 mice were subcutaneously injected on the right anterior flank with 5×10⁵ MC38-hPD-L1 tumor cells suspended in 0.1 mL PBS. The tumor-bearing mice whose average tumor size reached about 100 mm³ were randomly enrolled in the group hIgG 1 20 mg/kg, the group IMC-001 3 mg/kg, and the group IMC-001 10 mg/kg (each group consisting of 8 mice). All test substances were intraperitoneally administered to the tumor-bearing mice at a frequency of 3 times a week for 3 weeks.

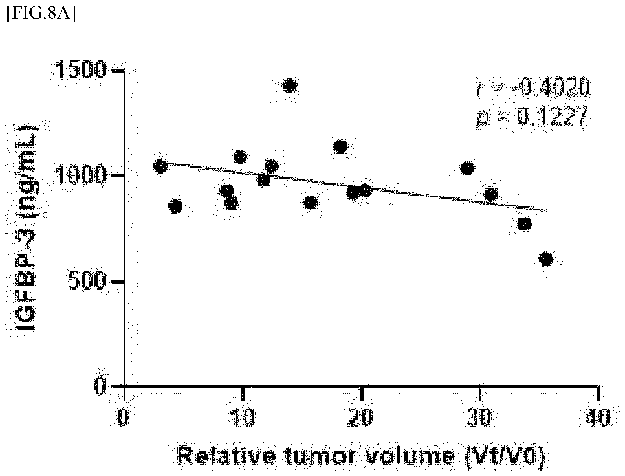

The mice were sacrificed on day 21. GraphPad Prism 8 software was used to plot graphs of tumor volume, and comparison between the groups was performed using two-way ANOVA with Dunnett's multiple comparison test (p-value; * <0.05, ** <0.01, *** < 0.001, **** <0.0001) (FIG. 8A).

### Example 3.2.2. Determination of mouse serum IGFBP-3 concentration by ELISA

At the time of sacrifice of the mice as in Example 3.2.1, all serum samples were collected and stored at -80°C until analysis.

Mouse serum IGFBP-3 was measured by enzyme-linked immunosorbent assay (ELISA) using the mouse IGFBP-3 duoset ELISA kit (DY775, R&D Systems, Minneapolis, Minnesota, United States), and the procedure was performed according to the instructions of the manufacturer of the kit. All samples were diluted at a ratio of 1:2,000 using 1× reagent diluent concentrate 3 from Duoset Ancillary Reagent Kit 3 (DY009, R&D Systems) within the range of the standard curve. Specifically, the detection antibody was diluted using 1× reagent diluent concentrate 3 containing 2% heat-inactivated normal goat serum (DY005, Reagent Additive 1, R&D Systems) according to the manufacturer's recommendations. All samples were run in duplicate.

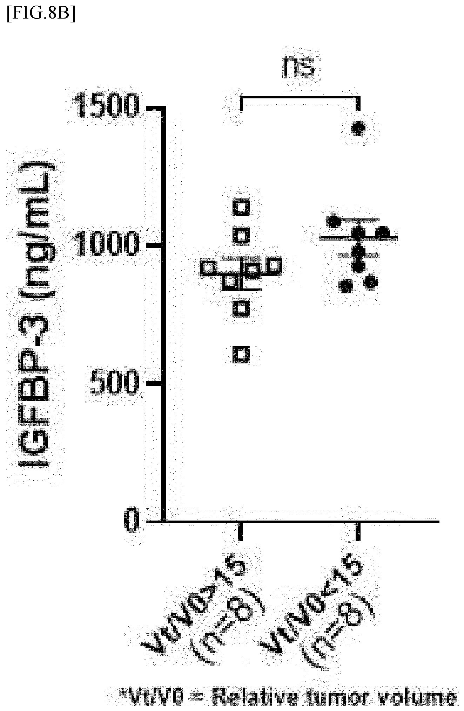

GraphPad Prism 8 software was used to plot variable data. Correlation between two parameters (serum IGFBP-3 and relative tumor volume) was identified using Pearson's correlation analysis (FIG. 8A). Comparison of differences between two groups was performed using an unpaired t-test, and a p-value of less than 0.05 was considered statistically significant (p-value; * <0.05, ** <0.01, *** <0.001, **** <0.0001) (FIG. 8B). In FIG. 8B, based on the relative tumor volume, the total of 16 mice administered with IMC-001 were divided into two groups of eight (8) each, that is, a group with large tumor growth (group having poor responsiveness (Vt/V0>15), n=8) and a group with small tumor growth (group having good responsiveness (Vt/V0<15), n=8), and IGFBP-3 was measured.

### Example 3.2.3. Changes in tumor volume following administration of IMC-001 in CT26-hPD-L1/hCD47 tumor model

In vivo efficacy of IMC-001 was evaluated for treatment of CT26-hPD-L1/hCD47 tumor in BALB/c mice as a colorectal cancer model.

For tumor development, the BALB/c mice were subcutaneously injected on the right anterior flank with 2×10⁶ CT26-hPD-L1/hCD47 tumor cells suspended in 0.1 mL PBS. The tumor-bearing mice whose average tumor size reached about 100 mm³ were randomly enrolled in the group hIgG 1 21.4 mg/kg, the group IMC-001 2.1 mg/kg, and the group IMC-001 10.7 mg/kg (each group consisting of 8 mice). All test substances were intraperitoneally administered to the tumor-bearing mice at a frequency of twice a week for 4 weeks.

The mice were sacrificed on day 28 after the first administration of IMC-001. GraphPad Prism 8 software was used to plot graphs of tumor volume, and comparison between the groups was performed using two-way ANOVA with Dunnett's multiple comparison test (p-value; * <0.05, ** <0.01, *** < 0.001, **** <0.0001) (FIG. 8A).

### Example 3.2.4. Determination of mouse serum IGFBP-3 concentration by ELISA

At the time of sacrifice of the mice as in Example 3.2.3, all serum samples were collected and stored at -80°C until analysis.

Mouse serum IGFBP-3 was measured by enzyme-linked immunosorbent assay (ELISA) using the mouse IGFBP-3 duoset ELISA kit (DY775, R&D Systems, Minneapolis, Minnesota, United States), and the procedure was performed according to the instructions of the manufacturer of the kit. All samples were diluted at a ratio of 1:2,000 using 1× reagent diluent concentrate 3 from Duoset Ancillary Reagent Kit 3 (DY009, R&D Systems) within the range of the standard curve. Specifically, the detection antibody was diluted using 1× reagent diluent concentrate 3 containing 2% heat-inactivated normal goat serum (DY005, Reagent Additive 1, R&D Systems) according to the manufacturer's recommendations. All samples were run in duplicate.

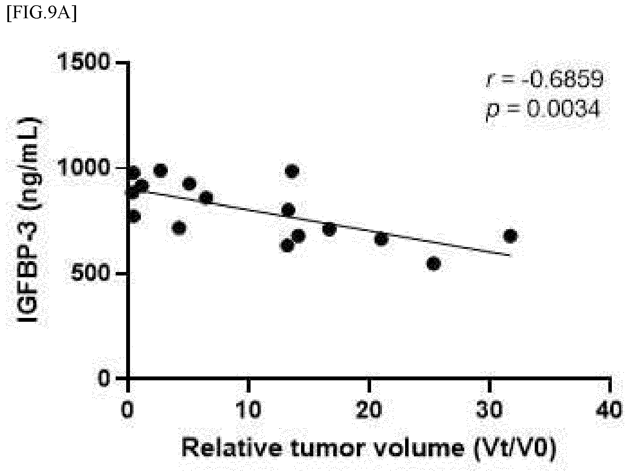

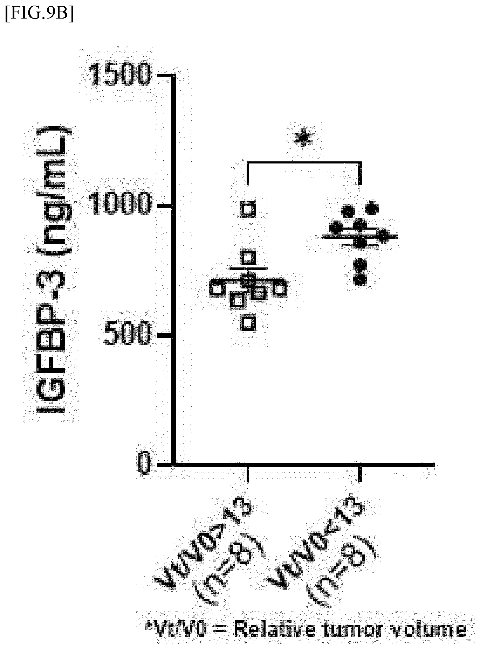

GraphPad Prism 8 software was used to plot variable data. Correlation between two parameters (serum IGFBP-3 and relative tumor volume) was identified using Pearson's correlation analysis (FIG. 9A). Comparison of differences between two groups was performed using an unpaired t-test, and a p-value of less than 0.05 was considered statistically significant (p-value; * <0.05, ** <0.01, *** <0.001, **** <0.0001) (FIG. 9B). In FIG. 9B, based on the relative tumor volume, the total of 16 mice administered with IMC-001 were divided into two groups of eight (8) each, that is, a group with large tumor growth (group having poor responsiveness (Vt/V0>13), n=8) and a group with small tumor growth (group having good responsiveness (Vt/V0<13), n=8), and IGFBP-3 was measured.

### Example 3.2.5. Results

In the two colorectal cancer mouse models (MC38 and CT26) used in Examples 3.2.1 to 3.2.4, tumor growth was inhibited in the groups administered with IMC-001 as compared with each control group (hIgG1) (FIGS. 8A and 9A).

In addition, as shown in FIG. 8B (average IGFBP-3 value for Vt/V0>15: 900.1 ng/mL (SEM value: 56.6); average IGFBP-3 value for Vt/V0<15: 1031.6 ng/mL (SEM value: 64.2)) and FIG. 9B (average IGFBP-3 value for Vt/V0>13: 713.1 ng/mL (SEM value: 46.3); average IGFBP-3 value for Vt/V0<13: 880.6 ng/mL (SEM value: 33.7)), in the groups (n=16) administered with IMC-001, serum IGFBP-3 level was significantly higher in the group with small tumor growth (group having good responsiveness, n=8) than the group with large tumor growth (group having poor responsiveness, n=8). In particular, the serum IGFBP-3 level was significantly high in the CT26 model.

From these results, since in the groups administered with IMC-001, a higher IGFBP-3 level was observed as a relative tumor volume was smaller, it was identified that IGFBP-3 could be used as a biomarker to predict whether an anti-PD-L1 antibody such as IMC-001 would exert a therapeutic effect.

## Claims

1. A composition for predicting therapeutic responsiveness of a cancer patient to a cancer immunotherapy, comprising an agent capable of measuring an expression level of mRNA or protein of one or more genes selected from the group consisting of OPN, POSTN, and IGFBP-3.

2. The composition of claim 1, wherein the one or more genes comprise OPN.

3. The composition of claim 1, wherein the one or more genes comprise OPN and POSTN.

4. The composition of claim 1, wherein the one or more genes comprise OPN, POSTN, and IGFBP-3.

5. The composition of claim 1, wherein the cancer immunotherapy inhibits binding between PD-L1 and PD-1.

6. The composition of claim 1, wherein the cancer immunotherapy is an anti-PD-L1 antibody.

7. The composition of claim 6, wherein the anti-PD-L1 antibody comprises a heavy chain comprising the amino acid sequence set forth in SEQ ID NO: 3 and a light chain comprising the amino acid sequence set forth in SEQ ID NO: 4.

8. The composition of claim 1, wherein the agent capable of measuring an expression level of mRNA is an antisense nucleotide, a primer, or a probe which complementarily binds to the gene.

9. The composition of claim 1, wherein the agent capable of measuring an expression level of protein is an antibody that specifically binds to the protein of the gene.

10. The composition of claim 1, wherein the cancer is a solid cancer.

11. The composition of claim 1, wherein the cancer is one or more selected from the group consisting of cholangiocarcinoma, colon cancer, adenocarcinoma, rectal cancer, breast cancer, synovial sarcoma, chondrosarcoma, thyroid cancer, stomach cancer, thymus cancer, cervical cancer, glioma, brain cancer, melanoma, lung cancer, bladder cancer, prostate cancer, leukemia, kidney cancer, liver cancer, colorectal cancer, pancreatic cancer, ovarian cancer, lymphoma, uterine cancer, oral cancer, bronchial cancer, nasopharyngeal cancer, laryngeal cancer, skin cancer, blood cancer, parathyroid cancer, and ureteral cancer.

12. A kit for predicting therapeutic responsiveness of a cancer patient to a cancer immunotherapy, comprising the composition of any one of claims 1 to 11.

13. A method for providing information for predicting therapeutic responsiveness of a cancer patient to a cancer immunotherapy, comprising:
measuring an expression level of mRNA or protein of one or more genes selected from the group consisting of OPN, POSTN, and IGFBP-3 in a biological sample obtained from the cancer patient; and
evaluating the patient's therapeutic responsiveness to the cancer immunotherapy, based on the measured expression level of mRNA or protein.

14. The method of claim 13, wherein the one or more genes comprise OPN.

15. The method of claim 13, wherein the one or more genes comprise OPN and POSTN.

16. The method of claim 13, wherein the one or more genes comprise OPN, POSTN, and IGFBP-3.

17. The method of claim 13, wherein the cancer immunotherapy inhibits binding between PD-L1 and PD-1.

18. The method of claim 13, wherein the cancer immunotherapy is an anti-PD-L1 antibody.

19. The method of claim 13, further comprising determining whether the patient needs to receive treatment with the cancer immunotherapy or needs to continue treatment with the cancer immunotherapy, depending on the evaluation of the patient's therapeutic responsiveness to the cancer immunotherapy.

20. The method of claim 13, wherein the expression level is measured before, after, or both before and after administration of the cancer immunotherapy.

21. The method of claim 20, wherein the expression level is measured before and after administration of the cancer immunotherapy, wherein when the gene is OPN or POSTN, the patient's therapeutic responsiveness is evaluated as being low in a case where the measured expression level is significantly higher after administration of the cancer immunotherapy than before administration of the cancer immunotherapy.

22. The method of claim 20, wherein the expression level is measured before administration of the cancer immunotherapy, wherein when the gene is OPN or POSTN, the patient's therapeutic responsiveness is evaluated as being low in a case where the measured expression level is significantly higher than an expression level thereof in other control cancer patients before administration of the cancer immunotherapy.

23. The method of claim 20, wherein the expression level is measured before and after administration of the cancer immunotherapy, wherein when the gene is IGFBP-3, the patient's therapeutic responsiveness is evaluated as being low in a case where the measured expression level is significantly lower after administration of the cancer immunotherapy than before administration of the cancer immunotherapy.

24. The method of claim 20, wherein the expression level is measured before administration of the cancer immunotherapy, wherein when the gene is IGFBP-3, the patient's therapeutic responsiveness is evaluated as being low in a case where the measured expression level is significantly lower than an expression level thereof in other control cancer patients before administration of the cancer immunotherapy.

25. The method of claim 13, wherein the expression level of mRNA is measured by one or more methods selected from the group consisting of in situ hybridization, reverse transcriptase polymerase chain reaction (RT-PCR), competitive reverse transcriptase polymerase chain reaction (competitive RT-PCR), real-time reverse transcriptase polymerase chain reaction (real-time RT-PCR), RNase protection assay (RPA), microarray, and Northern blotting.

26. The method of claim 13, wherein the expression level of protein is measured by one or more methods selected from the group consisting of Western blotting, radioimmunoassay (RIA), radioimmunodiffusion, enzyme-linked immunosorbent assay (ELISA), immunoprecipitation, flow cytometry, immunofluorescence, Ouchterlony, complement fixation assay, and protein chip.

27. A method for treating cancer based on therapeutic responsiveness of a cancer patient to a cancer immunotherapy, comprising:
(i) administering the cancer immunotherapy to the cancer patient; and
(ii) measuring an expression level of mRNA or protein of one or more genes selected from the group consisting of OPN, POSTN, and IGFBP-3 in a biological sample obtained from the cancer patient, before, after, or both before and after administration of the cancer immunotherapy.
